# EUROPEAN PATENT APPLICATION

(11) **EP 1 707 166 A1**
(43) Date of publication of application: **04.10.2006**
(21) Application number: 06111287.6
(22) Date of filing: 16.03.2006
(51) Int. Cl.: A61F 9/007, A61M 3/02, A61B 17/32

(54) **Irrigation tip**

(30) Priority: 28.03.2005 US 91214; 11.07.2005 US 178903
(71) Applicant: Alcon, Inc., 6331 Hünenberg (CH)
(72) Inventor: Ghannoum, Ziad R., Trabuco Canyon, CA 92679 (US)
(74) Representative: Hanna, Peter William Derek

(57) **Abstract**

An irrigation tip for a surgical handpiece is provided, having a proximal hollow shaft portion (12) of generally circular cross-section, and a distal hollow shaft portion (14) of generally oval or elliptical cross-section. This provides a flattened or oval open distal end (28) on the distal shaft portion. The shaft may be straight or curved. The tip is useful in fitting into the anterior chamber angle of the eye, when practising the effective delivery of pulses of a balanced salt solution to the trabecular meshwork during glaucoma surgery.

## Description

### Background of the Invention

This invention relates generally to the field of eye surgery and more particularly to a method for glaucoma surgery.

Glaucoma affects approximately 2% of the population under 65 years of age and 11% over 65, and it is exceedingly difficult to diagnose and define. The eye is a hollow structure that contains a clear fluid called "aqueous humor." Aqueous humor is formed in the posterior chamber of the eye by the ciliary body at a rate of about 2.5 microliters per minute. The fluid, which is made at a fairly constant rate, then passes around the lens, through the pupillary opening in the iris and into the anterior chamber of the eye. Once in the anterior chamber, the fluid drains out of the eye through three different routes. Fluid can be absorbed by the iris, which normally accounts for less than 1 % of drainage. In the "uveoscleral" route, fluid percolates between muscle fibers of the ciliary body. This route accounts for less than percent of the aqueous outflow in humans. The primary pathway for aqueous outflow in humans is through the "canalicular" route that involves the trabecular meshwork and Schlemm's canal.

The trabecular meshwork and Schlemm's canal are located at the junction between the cornea and the sclera. This is anterior to the insertion of the iris into the scleral. The junction or corner of the iris insertion and the cornea is called "the angle." The trabecular meshwork is a wedge-shaped structure that runs around the circumference of the eye. It is composed of collagen beams arranged in a reedimensional sieve-like structure. The beams are lined with a monolayer of endothelial. cells called trabecular cells. The spaces between the collagen beams are filled with an extracellular substance that is produced by the trabecular cells. These cells also produce enzymes that degrade the extracellular material. Schlemm's canal is adjacent to the trabecular meshwork. The outer wall of the trabecular meshwork coincides with the inner wall of Schlemm's canal. Schlemm's canal is a tube-like structure that runs around the circumference of the cornea. In human adults, Schlemm's Canal is believed to be divided by septa into a series of autonomous, dead-end canals.

The aqueous fluid travels through the spaces between the trabecular beams, across the inner wall of Schlemm's canal into the canal, through a series of collecting channels that drain from Schlemm's canal and into the episcleral venous system. In a normal situation, aqueous production is equal to aqueous outflow and intraocular pressure remains fairly constant with a mean of approximately 16 mm Hg. In glaucoma, the resistance through the canalicular outflow system is abnormally high, creating a higher intraocular pressure.

In primary open angle glaucoma, the most common form of glaucoma in the United States, the abnormal resistance is believed to be along the outer aspect of trabecular meshwork and the inner wall of Schlemm's canal. In normals, this accounts for approximately 50% of resistance. In glaucoma patients, this accounts for all of the additional resistance. It is believed that an abnormal metabolism of the trabecular cells might lead to an excessive build up of extracellular materials or a build up of abnormally "stiff' materials in this area. Histopathology of glaucoma eyes also demonstrates a collapse of Schlemm's, canal. Primary open angle glaucoma accounts for approximately eighty-five percent of all glaucoma in the Americas and Europe. Other forms of glaucoma (such as angle closure glaucoma and secondary glaucomas) also involve decreased outflow through the canalicular pathway but the increased resistance is from other causes such as mechanical blockage, inflammatory debris, cellular blockage, etc.

With the increased resistance, the aqueous fluid builds up because it cannot exit fast enough. As the fluid accumulates, the intraocular pressure (IOP) within the eye increases. The increased IOP compresses the axons in the optic nerve and also may compromise the vascular supply to the optic nerve. The optic nerve carries vision the eye to the brain. Some optic nerves seem more susceptible to a specific level of IOP than other eyes. While research is investigating ways to protect the nerve from an elevated pressure, the only therapeutic approach currently available in glaucoma that is proven either to prevent or retard the progression of nerve loss and resultant visual disability leading to blindness is to reduce the intraocular pressure.

The clinical treatment of glaucoma is commonly approached in a step-wise fashion. Medication often is the first treatment option. Usually administered either topically, these medications work to either reduce aqueous production or they act to increase outflow. Currently available medications have many serious systemic side effects including: congestive heart failure, respiratory distress, systemic hypotension, depression, sedation, renal stones, aplastic anemia, sexual dysfunction and death. As an asymptomatic disease, compliance with medical therapy is a major problem, with estimates that over half of glaucoma patients do not follow their correct dosing schedules. This lack of adherence to prescribed medical therapy may account for the fact that more than 20% of patients go blind bilaterally within a 20 year period.

When medication fails to adequately reduce the pressure, laser trabeculoplasty often is performed. In laser trabeculoplasty, energy from a laser is applied to a number of noncontiguous spots in the trabecular meshwork. It is believed that the laser energy stimulates the metabolism of the trabecular cells in some way, and changes the extracellular material in the trabecular meshwork. In approximately eighty percent of patients, aqueous outflow is enhanced and IOP decreases. However, the effect often is either not sufficient or not long lasting and at least fifty percent of patients develop an elevated IOP within five years. In many cases, the laser surgery is not usually repeatable. In addition, laser trabeculoplasty is not an effective treatment for primary open angle glaucoma in patients less than fifty years of age, nor is it effective for angle closure glaucoma and many secondary glaucomas.

If laser trabeculoplasty does not adequately reduce the IOP, then filtering surgery is performed. With filtering surgery, a hole is made in the sclera near the angle. This hole allows the aqueous fluid to leave the eye through an alternate route. The most commonly performed filtering procedure is a trabeculectomy. In a trabeculectomy, a conjunctiva incision is made, the conjunctiva being the transparent tissue that covers the sclera. The conjunctiva is moved aside, exposing the sclera at the limbus. A partial thickness scleral flap is made and dissected half-thickness into the cornea. The anterior chamber is entered beneath the scleral flap and a section of deep sclera and/or trabecular meshwork is excised. The scleral flap is loosely sewn back into place. The conjunctival incision is tightly closed. Post-operatively, the aqueous fluid passes through the hole, beneath the scleral flap which offers some resistance and collects in an elevated space beneath the conjunctiva called a bleb. The fluid then is either absorbed through blood vessels in the conjunctiva or traverses across the conjunctiva into the tear film.

Trabeculectomy and filtration, surgery are both associated with many problems. Fibroblasts that are present in the opisclera proliferate and migrate, and can scar down the scleral flap. Failure from scarring may occur, particularly in children, young adults, those with active inflammation or eyes with prior intraocular surgery. Of eyes that have an initially successful trabeculectomy, up to eighty percent will fail from scarring within three to five years after surgery. To minimize this scarring, surgeons now are applying antifibrotic agents such as mitomycin C (MMC) and 5-fluorouracil (5-FU) to the scleral flap at the time of surgery or giving injections of 5-FU daily for up to 14 days postoperatively. The use of these agents has increased the success rate of trabeculectomy but also has increased the prevalence of multiple complications which are sight-threatening and potentially blinding. The most serious complication is cataract which can occur in up to 20% of eyes. Bleb infections can occur in all eyes for the rest of the patient's life following surgery. Hypotony is a problem that develops when aqueous flows exits the eye faster than aqueous humor is made. The eye pressure drops too low (usually less than 6.0 mmHg); the structure of the eye collapses and vision decreases as the choroids and macula become swollen and folded.

Trabeculectomy also creates a pathway for aqueous fluid to escape to the surface of the eye. At the same time, it creates a pathway for bacteria that normally live on the surface of the eye and eyelids to get into the eye. If this happens, an internal eye infection can occur called endophthalmitis. Endophthalmitis often leads to permanent and profound visual loss. Endophthalmitis can occur anytime after trabeculectomy. The risk increases with the thin blebs that develop after MMC and 5-FU and is cumulative at about 0.4% per year. Another factor that contributes to infection is the placement of a bleb. Eyes that have trabecalectomy performed inferiorly have about eight times the risk of eye infection than eyes that have a superior bleb. Therefore, initial trabeculectomy is performed superiorly under the eyelid, in either the nasal or temporal quadrant.

In addition to scarring, hypotony and infection, there are other complications of trabeculectomy. The bleb can tear and lead to profound hypotony. The bleb can be irritating and can disrupt the normal tear film, leading to vision Approximately 67% of patients experience continuous persistent discomfort which can last for years. Patients with blebs generally cannot wear contact lenses. All of the complications from trabeculectomy stem from the fact that fluid is being diverted from inside the eye to the external surface of the eye.

Recently, a surgical technique has been suggested wherein pulses of relatively high pressure irrigating balanced salt solution are directed at the trabecular meshwork. These pulses can be focused, thereby perforating the trabecular meshwork, car applied over a larger area so as to stimulate the trabecular meshwork for improved fluid transport. In addition, the pulses of the irrigating fluid may clean away material, such as iris pigment, that may be blocking or clogging the trabecular meshwork, Existing irrigating tips, however, are not optimized for this technique.

Therefore a need continues to exist for an irrigating tip for use with surgical methods for increasing the outflow of aqueous from the eye to help reduce IOP in glaucoma patients.

### Brief Summary of the Invention

The inventor of the present invention has discovered that an irrigating tip having a flattened or oval shape more easily fits into the anterior chamber angle, thereby allowing more effective delivery of the pulses of balanced salt solution to the trabecular meshwork. The invention provides an irrigation tip in accordance with claims which follow,

Accordingly, one objective of the present invention is to provide an irrigating tip for practicing a surgical method for treating glaucoma.

Another objective of the present invention is to provide an irrigating tip for practicing a surgical method for increasing the outflow out of the eye through the trabecular meshwork.

These and other advantages and objectives of the present invention will become apparent from the detailed description and claims that follow.

### Brief Description of the Drawings

FIG. 1 is an enlarged perspective view of the tip of the present invention being used during ophthalmic surgery.
FIG. 2 is an exploded perspective view of the tip of the present invention.

### Detailed Description of the Invention

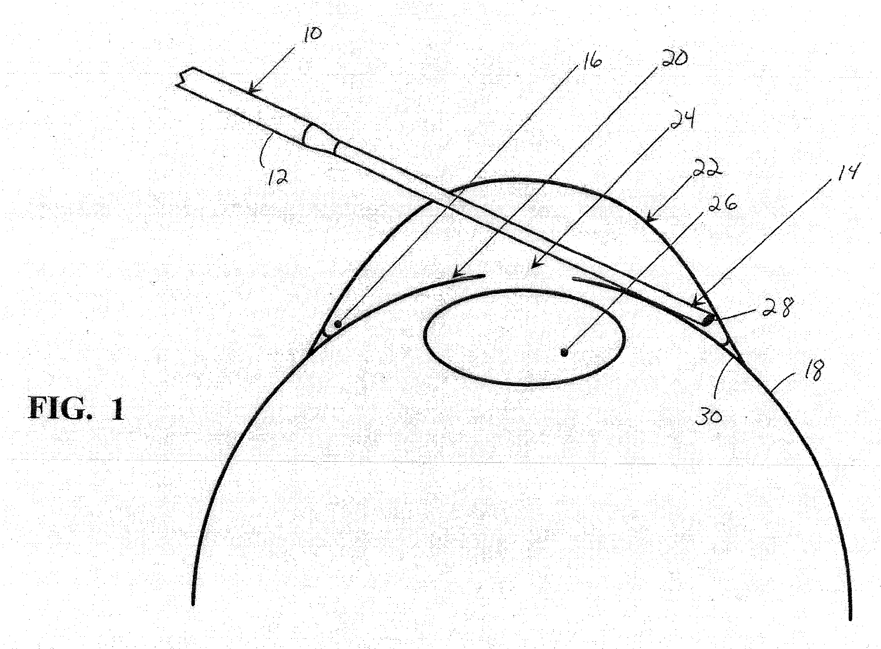

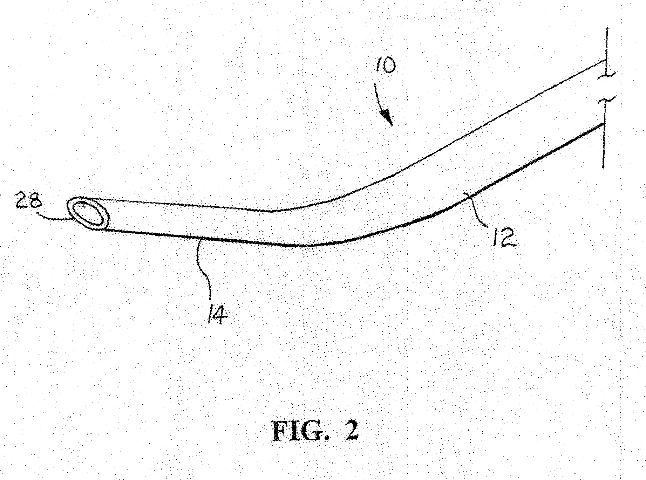

As best seen in FIG. 1, tip 10 of the present invention generally includes proximal shaft 12 and distal shaft 14. Tip 10 may be used with any suitable handpiece (not shown), such as those handpieces disclosed in U.S. Patent No, 6,575,929 (Sussman, et al.), U.S. Patent No. 5,322,504 (Doherty, et al.) and U.S. Patent No. 5,562,692 (Bair) and commercially available from sources such as Alcon Laboratories, Inc., Fort Worth, Texas and sold under the trademark AQUALASE®. Proximal shaft 12 preferably is round, and distal shaft 14 preferably is flattened into an elliptical or oval shape, as best seen in FIG. 2. Proximal shaft 2 preferably has an outer diameter of between approximately 0,039 inches and 0.041 inches. Distal shaft 14 preferably has a major outer diameter of between approximately 0.028 inches and 0.032 inches and a minor outer diameter of between approximately 0.14 inches and 0.18 inches. Tip 10 is preferably made from stainless steel or titanium, but other materials may also be used. Tip 10 preferably has an overall length of between 1.55 inches and 1.75 inches, with around 1,65 inches being most preferred. Tip 10 may be formed using conventional metalworking technology and preferably is electropolished to remove any burrs and may be straight, as shown in FIG. 1 or curved, as shown in FIG. 2.

In use, tip 10 is most suitable for irrigating anterior chamber angle 16 of eye 18. Angle 16 is located at the intersection of iris 20 and cornea 22. As points of reference, other structures of the eye include pupil 24 and lens 26. In particular, tip 10 is useful in delivering pulses of irrigating fluid to angle 16 to assist in cleansing or opening trabecular meshwork 30 which is locating at angle 16. Pulses of fluid will be directed out of distal end 28 of distal shaft 14 and toward angle 16. The fluid pulses can be focused, thereby perforating trabecular meshwork 30, or applied over a larger area so as to stimulate trabecular meshwork, 30 for improved fluid transport. In addition, the pulses of the irrigating fluid can clean or clear away material, such as iris pigment, that may be blocking or clogging trabecular meshwork 30. The flattened or oval shape of distal shaft 14 allows the surgeon to place distal end 28 more closely to angle 16 than prior art round tips.

This description is given for purposes of illustration and explanation. It will be apparent to those skilled in the relevant art that changes and modifications may be made to the invention described above without departing from its scope,

## Claims

1. An irrigation tip (10), comprising:
a) a proximal hollow shaft portion (12) of generally circular cross-section;
b) a distal hollow shaft portion (14) of generally oval or elliptical cross-section, and being fluidly connected to the proximal shaft portion.; and
c) an open distal end (28) on the distal shaft portion.

2. The irrigation tip of claim 1, wherein the shaft is straight.

3. The irrigation tip, wherein the shaft is curved.

4. The irrigation tip of any of claims 1 to 3, wherein the proximal shaft portion (12) has an outer diameter of between. 1.0 mn (0.039 inches) and 1,04 mm (0.041 inches), and the distal shaft portion (14) has a major outer diameter of between 0.71 mm (0.028 inches) and 0.81 mm. (0.032 inches) and a minor outer diameter of between 3.56 mm (0. 14 inches) and 4.57 mm (0.18 inches).
